# EUROPEAN PATENT SPECIFICATION

(11) **EP 0 629 135 B1**
(45) Date of publication and mention of the grant of the patent: **26.07.2000**
(21) Application number: 93907173.4
(22) Date of filing: 02.03.1993
(51) Int. Cl.: A61L 2/18

(54) **VIRAL INACTIVATION METHOD**
VERFAHREN ZUR INAKTIVIERUNG VON VIREN
PROCEDE D'INACTIVATION VIRALE

(30) Priority: 02.03.1992 US 844513
(43) Date of publication of application: 21.12.1994
(73) Proprietor: BIOENG, INC., Woburn, MA 01801 (US)
(72) Inventor: CASTOR, Trevor, P., Arlington, MA 02174 (US); LANDER, Arthur, D., Boston, MA 02109 (US)
(74) Representative: Deans, Michael John Percy
(86) International application number: PCT/US93/01916
(87) International publication number: WO 93/17724

(56) References cited:
- WO-A-85/05273
- WO-A-87/02697
- WO-A-91/01367

## Description

This invention relates to methods for the inactivation of viruses.

Infectious viruses, unless inactivated, can be readily transmitted from biological products derived from human plasma as well as recombinant-DNA and monoclonal antibody sources. The hepatitis B virus (HBV) as well as the agents of non-A and non-B hepatitis (NANBHV) have plagued patients, physicians and manufacturers for many years. In recent years, the human immunodeficiency virus (HIV) -- the etiologic agent of acquired immunodeficiency syndrome (AIDS) -- has become a very serious and epidemic problem. In several instances, coagulation factor concentrates have transmitted HIV to hemophiliacs, some of whom have subsequently developed AIDS (an estimated 65 % of the 20,000 hemophiliacs in the United States are infected with HIV, Wall Street Journal, 27th December 1990. Morgenthaler, a leading researcher in this field, states in his preface to Viral Inactivation in Plasma Products in Clinical Studies in Hematology and Blood Transfusion, Edited by A. Hassig *et al*, Karger, New York (1989) that: "Clearly, there is a need for methods that inactivate viruses in blood, without causing harm to the components intended for therapeutic use."

Viral inactivation of recombinant-DNA products is still a major and active concern for the biotechnology and pharmaceutical industries as well as the regulatory agencies. The FDA in Points to Consider in the Manufacture and Testing of Monoclonal Antibody Products for Human Use states that: "It is strongly recommended that validated procedures ... which remove and/or inactivate viruses and DNA if present be employed during purification..." For reasons of safety and public health, the quest is to discover and develop processes for inactivating viruses without denaturing the biologically active products which are commonly labile and quite sensitive to conventional viral inactivation techniques.

A number of approaches have been employed for the inactivation of viruses in therapeutic proteins derived from human plasma and other biologically active raw materials. Some of these approaches include: heating or pasteurization; chemical inactivation agents; ultraviolet (UV) and gamma irradiation; solvent extraction of essential lipids; neutralization with specific antibodies; and removal by various fractionation techniques.

Several heat treatment methods have been invented to inactivate viruses without adversely affecting the biological activity of particular proteins. Most of these methods require the use of stabilizers to protect the proteins or require the heat treatment of the materials in their lyophilized state. Fernandez et al. (U.S. Pat. No. 4,440,679) utilize carbohydrate stabilizers for the pasteurization of Factor VIII which is a very thermally labile plasma protein. Reubenstein (U:S. Pat. No. 4,456,590) shows that Factor VIII can be pasteurized at 60C for 10 hours if first lyophilized and Thomas in U.S. Pat. No. 4,495,278 shows the same for Factor IX. Mitra et al. (U.S. Pat. No. 4,762,714) show viral inactivation in an immune globulin product by controlling conditions of pH, temperature and time. Feldman et al. (U.S. Pat. No. 4,876,241) teach a method of stabilizing biological and pharmaceutical products with mixtures of sugar and sugar alcohols with salts of sulfate and acetate prior to thermal inactivation of viral and bacterial contaminants.

The current standard for rendering human serum albumin free from hepatitis virus (both HBV and NANBHV) is pasteurization at 60°C for 10 hours. High concentrations of stabilizers (such as citrate, sucrose and caprylate) are typically used to protect biological activity during pasteurization. Plasma fractions such as Factor VIII can also be pasteurized in the lyophilized state which requires heating the plasma powder at 60°C in the presence of stabilizers for more than 72 hours. The major problems with pasteurization are: long processing times; inactivation of plasma proteins and fractions; and the use of high concentrations of stabilizers which must be removed before therapeutic use (stabilizers also tend to protect the viruses as well as the proteins, thereby requiring longer and harsher treatment conditions).

Recently, Charm et al. in "High Temperature - Short Time Heat Inactivation of HIV and other Viruses in Human Blood Plasma," supported in part by NHLBI Grant No. HL41221 to the New York Blood Center, Nov. 16, 1990 and accepted for publication in Vox Sanguinis, 1991 have reported a high temperature-short time (HTST) heat inactivation method for HIV and other viruses in human blood plasma without the use of stabilizers or freeze-drying. While the inactivation data is quite encouraging, further studies are required including the validation of hepatitis B and hepatitis C virus inactivation. The HTST technique, under conditions required to inactivate certain viruses, may be burdened by significant loss of biological activity. Additionally, this microwave technique may not be able to handle very viscous materials and may not be very scalable. HTST equipment will require the use of very small diameter tubes in order to achieve rapid-heat up rates and short residence times necessary for maintaining protein integrity.

Chemical methods to inactivate viruses have been used by themselves or in combination with other physical separation methods: copper phenathroline and related compounds (Lembach, U.S. Pat. No. 4,534,972); tri-n-butyl phosphate and related compounds (Horowitz, U.S. Pat. No. 4,481,189); sodium caprylate in combination with heat and amino acids (Naito et al., U.S. Pat. No. 4,446,134); caprylic acid at a nonionized concentration (Seng et al., U.S. Pat. No. 4,939,176); Beta-propionolactone with colloidal silica adsorption (U.S. Pat. No. 4,370,264); combinations of phenol and formaldehyde for inactivating HIV virus in blood and blood components (Louderback, U.S. Pat. No. 4,833,165); mixtures of detergent, alcohol and ether (Prince, U.S. Pat. No. 4,591,505); di- or tri-alkylphosphate and detergent (Neurath et al., U.S. Pat. No. 4,764,369); a variety of techniques using other surfactants (U.S. Pat. Nos. 4,314,997; 4,315,919; and 4,613,501). Chemical sterilization techniques, such as the 1,10-phenanthroline-cuprous ion complex being developed by Cutter Biological, Division of Miles, Inc., Berkeley, CA, are still under development and will probably be plagued by chemical removal, residual toxicities and normal cell damage control (these chemical techniques work by degrading nucleic acids).

A variety of organic solvent techniques with/without the use of detergents has been devised. Treatment with halohydrocarbons is presented in U.S. Patent No. 4,511,556, and various alkanes, ketones and perfluorocarbons are suggested in U.S. Pat. No. 4,490,361. Organic solvent/detergent mixtures (such as ethyl ether/Tween 80, amyl acetate/deoxycholate and tri (n-butyl) phosphate/sodium cholate) are being developed. These techniques are burdened with: processing problems associated with removing the organic solvent and the detergent from the end-product; residual toxic chemicals in the blood plasma proteins and fractions; and ineffectiveness against viruses such as polio with negligible lipid content in their viral coat.

UV irradiation, developed during World War II, often fails to remove the infectivity of HBV and pathogens which exhibit very small cross-sections for inactivation; UV irradiation also results in protein denaturation and DNA damage to normal cells. In Europe, UV irradiation is also being used in conjunction with B-propionolactone which is considered a carcinogenic agent.

Several companies are developing microfiltration, traditionally used for the removal of bacteria from liquid streams, for virus removal from recombinant-DNA and monoclonal antibody products. Pall Ultrafine Filtration Company has developed a 0.04 micron (40 nanometers (nm)) hydrophilic filter for removing viruses and particles from such products. While this filter has been shown to be effective against viruses ranging in size from 80 to 120 nm, no consistent test has been made available for viruses less than 70 nm in size and very small viruses (such as polio with a diameter of 25 nm) are not expected to be effectively removed (Toth, C.S.: "Retention of Viral Contaminants by 0.04 micron ND filters," presented at the Engineering Conference, Miami, FL, 1990). Millipore Corporation, Bedford, MA has recently announced (June, 1991) the development of a nonselective filtration system for removing viruses from protein solutions. They claim 4-log removal of viruses greater than or equal to 28 nanometers in size and greater than 7-log removal of retroviruses (100 nanometers) with greater than 90 % recovery of proteins with a molecular weight less than 70,000. Millipore also announced that they are developing other membranes for biotherapeutics such as monoclonal antibodies and serum-based products. Some of the problems associated with microfiltration are: ineffectiveness against small viruses; nonspecific adsorption and loss of product; denaturation due to shear forces; excessive foaming and pore plugging; and difficulty to scale with the most significant disadvantage being the inability to inactivate viruses since a 1 % inefficiency of removal can cause subsequent product contamination.

Thus, there still exists substantial difficulty in inactivating viruses in the presence of thermally labile and sensitive proteins without utilizing additives which may be carcinogenic, toxic and/or damaging to biologically active compounds. These additives, which must be removed in a sterile post-process step, place an additional cost and time burden on conventional viral inactivation techniques.

Taniguchi *et al* "Sterilization of Plasma Powder by Treatment with SCF CO2", Agric. Biol. Chem., 51(12), 3425-3426, 1986, described a process for sterilizing plasma powder using treatment with supercritical carbon dioxide. Removal of viruses was never mentioned, and the focus of the reference was on testing for the removal of bacteria.

WO 91/01367 of BioEng, Inc describes the use of critical fluids for the disruption of microbial cells such as bacteria and yeast. WO 87/02697 suggests that critical fluids may be used for extracting a wide variety of biological materials, including the extraction of whole viruses from biological substrates. WO 85/05273 describes the use of halo hydrocarbons saturated with water for extracting lipids from viruses in dry material, thereby leading to their inactivation. The experiments described in WO 85/05273 show that if the degree of saturation is increased, near complete inactivation of virus can be achieved at the penalty of loss of activity of the accompanying proteinaceous material while if the activity of the proteinaceous material was to be maintained, the virus may not be completely inactivated. Prior to the present invention there was therefore a continuing need for a method of inactivating viruses particularly when they may be associated with proteinaceous products without incurring substantially denaturation.

In accordance with the present invention, there is provided a method of rendering a virus associated with a material inactive characterized in comprising the steps of:
a) contacting the material with a critical or near critical fluid at a pressure of at least 1000 psig (a gauge pressure of 6.89x10⁶N/m²), more preferably about 3000 psig (a gauge pressure of 2.068x10⁷N/m²), said critical or near critical fluid being capable of being received by said virus and being such that, upon removal of the critical or near critical fluid, said virus is inactivated; and
b) removing said critical or near critical fluid from said material and said virus.

It will become clear from the description set out below that we have discovered that critical fluids can be effective in inactivating viruses, including viral-like particles, but especially enveloped or lipid-coated viruses, without substantial denaturation of proteinaceous product and loss of biological activity.

We describe hereinbelow the steps of contacting a material with a critical fluid, separating the material from the critical fluid, and isolating the material under aseptic conditions. The material to be treated may be a solid, attached to a solid or in solution and preferably the material is contacted with the critical fluid for a sufficient period of time to render the material substantially virus free. In one preferred application, the material includes desired biological molecules having a desired activity and the material is contacted with the critical fluid under conditions such that the biological molecules substantially retain that activity.

Preferred temperatures range from 0.5 Tr to 2.0 Tr (where Tr, the REDUCED TEMPERATURE, is the absolute operating temperature divided by the absolute critical temperature) and most preferably is between 4°C and 40°C. It also is preferable that the material be subjected to the supercritical or near-critical fluids under pressures ranging from 0.2Pr to 20.0 Pr (where Pr, the REDUCED PRESSURE, is the operating pressure divided by the critical pressure) and most preferably in the range of 0.5Pr to 10.0Pr.

Utilizing a mouse retrovirus as a model for the AIDS virus, it has been demonstrated that the critical fluid viral inactivation process has the potential to inactivate enveloped viruses under remarkably mild conditions. We have also discovered that the process can be operated at relatively low temperatures and for comparatively short residence times with negligible denaturation of blood proteins and other beneficial plasma constituents. We have achieved as much as a 4-log reduction in viral activity in times as short as five minutes. Our processes are also amenable to continuous flow operation and readily scalable.

Potential applications include viral inactivation of plasma and its fractions, serum, recombinant-DNA therapeutics, vaccines and biomedical and research instruments and devices. Novel apparatus also are provided for carrying out the foregoing processes.

In the drawings:
Fig. 1 is a schematic illustration of an embodiment of apparatus suitable for putting a method in accordance with this invention into effect.
Fig. 2 shows HPLC chromatograms of various extracts of Baker's yeast using different extraction procedures.
Fig. 3 shows HPLC chromatograms of various extracts of Bakers yeast using different extraction procedures.
Fig. 4 shows HPLC chromatograms of various extracts of Baker's yeast using different extraction procedures.

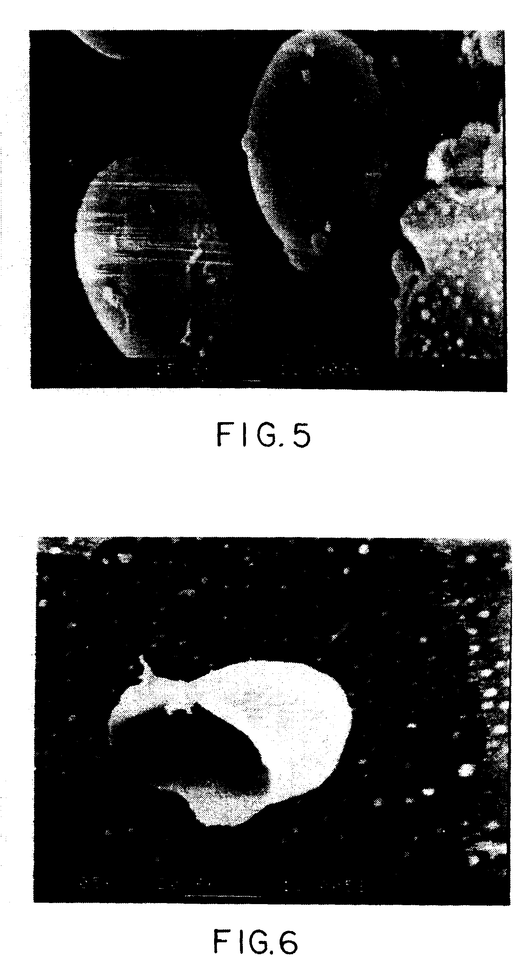

Fig. 5 is a scanning-electron micrograph showing a yeast cell prior to critical fluid treatment (magnification of 15,600 x).
Fig. 6 is a scanning-electron micrograph showing a single yeast cell after critical fluid treatment (magnification of 20.600x)

We describe herein a process for inactivating viruses and other particle-like pathogens using critical fluids. Such particle-like pathogens are intended to be encompassed by the term "virus" herein. By critical fluid it is meant a fluid that is at supercritical conditions. Also included are fluids that are at near (e.g. slightly below) supercritical conditions, and particularly fluids at or slightly below critical temperature and at or substantially above critical pressure. Preferred fluids are those that are gases at ambient conditions and that have critical temperatures of between 0°C and 100°C, most preferably between 0°C and 60°C. When working with biological material (e.g. proteinaceous material), fluids with critical temperatures of substantially less than 60°C (e.g. 40°C) are preferred so as to further preserve biological activity. Preferred fluids includes fluorocarbons such as chlorodifluoromethane, alkanes such as ethylene, propane and ethane, and binary fluids such as nitrous oxide and carbon dioxide. These fluids can be used with small quantities of polar entrainers or modifiers, known in the art as cosolvents. Cosolvents include substances such as ethanol, methanol, acetone, and ethylene glycol. Such cosolvents can effect inter alia the polarity of the critical fluid, thereby enhancing the capacity of the critical fluid to exactivate virus in certain materials.

The materials to be treated may be any material that can be combined with a selected critical fluid without substantially adversely affecting the material in its arena of intended use. Thus, the material may be a solid, nonorganic material, a solid organic material, a suspension or a solution. Our methods are particularly applicable to material having biological activity and particularly to proteinaceous materials attached to a solid support matrix or contained in a solution. Examples include blood plasma, blood plasma fractions, serum used for mammalian cell culture and solutions containing recombinant-DNA derived proteins. The protein even may be contained in living cells although typically the material treated is noncellular. By noncellular it is meant a material that is substantially free of prokaryotic or eukaryotic cells and in particular free of genetically-engineered or hybrid cell lines and the like adapted for producing a biological material of interest. An inconsequential amount of cells may be present in "noncellular" material where, for example, a pretreatment step for removing cells (e.g. centrifugation or cell disruption) has been less than 100% effective. The process also has applicability to the inactivation of viruses and other particle-like pathogens in semi-solid and solid matrices which are labile such as foods, spices, pharmaceutical powders and tablets, biomedical devices and membranes such as dialysis membranes.

As shown below, different fluids exhibit different degrees of success in inactivating viruses. As will be recognized by one of ordinary skill in the art, a particular fluid will be selected at least based upon at least two factors. Firstly, the fluid selected must be capable of inactivating virus to the desired degree. In certain instances, inactivation of a particular virus may be the goal, and the fluid should be selected accordingly. Secondly, the fluid should be selected such that it does not damage, inactivate or otherwise adversely affect the material being treated in the arena of intended use for that material. For example, in the case of a protein, a fluid having a critical temperature substantially below 60°C and that does not tend to chemically denature the protein or otherwise adversely interact with the protein is preferred.

It further will be understood by one of ordinary skill in the art that the particular fluid selected as well as the time of exposure of the critical fluid to the material, the temperature of the mixture and the pressure of the mixture are interdependent and together or individually may determine the appropriate conditions for the desired result. For example, the time of exposure of the material to the critical fluid may affect the degree of viral inactivation. Thus, conditions for treating the material include those of sufficient time exposure to ensure that the material is substantially free of virus. By substantially free of virus it is meant a reduction in virus below that which is detectable by the procedures described herein. The time of exposure as well as the temperature together may affect the biological activity of a treated, isolated material. In connection with proteins, it is preferred to select conditions such that the desired biological activity of the protein is substantially retained. By substantially retained it is meant that at least 50% of the desired biological activity is retained. As described in the summary, the material is separated from the critical fluid under aseptic conditions. In the case of a solution containing for example a protein, to accomplish separation, the mixture is decompressed thereby resulting in a phase separation of the fluid from the solution containing the proteinaceous product. The material then is isolated under aseptic conditions.

By isolating the material it is meant separating the material from the equipment such as into a sterile bottle or a container (e.g. a flask) or into a package such as a hermetically sealed package. It does not include simply separating the material from the critical fluid into a compartment or container that is part of the sterilization equipment itself. It should be understood, nevertheless, that the containers or packages used in isolating the material may be at least temporarily attachable to the equipment so as to facilitate transfer of the material from the equipment to its isolated state in a sterile container or package. The treated material also may be first collected from the equipment and then filtered such as through a filter to remove bacteria in order to isolate the material under aseptic conditions.

The apparatus includes a source of fluid, a high-pressure, recirculation loop, a separation chamber, and at least one low pressure trap. Viral inactivation occurs in the high-pressure recirculation loop, which is rated for continuous operation at 5,000 psig (a gauge pressure of 3.447x10⁷N/m²) and 100°C. The high-pressure recirculation loop consists of: a chamber 10 into which the material to be treated and the critical fluid are introduced: an injection port 12 for introducing the product into the soaking chamber; a static in-line mixer 14 for continuously mixing the mixture of product and fluid; and a circulation pump 16 for moving the mixture to the in-line mixer 14; two thermocouples, one associated with the separation chamber (thermocouple T1) and the other located just upstream of the in-line mixer (thermocouple T2); a pressure indicator P2 also located just upstream of the static in-line mixer; and various interconnecting lines 18, 20, 22, 24, 26, 28, 30, 32, 34 and 36 as well as several process valves PV-3, PV-4, PV-5, PV-6 and PV-7 for chanelling and controlling the movement of fluid throughout the high-pressure recirculation loop.

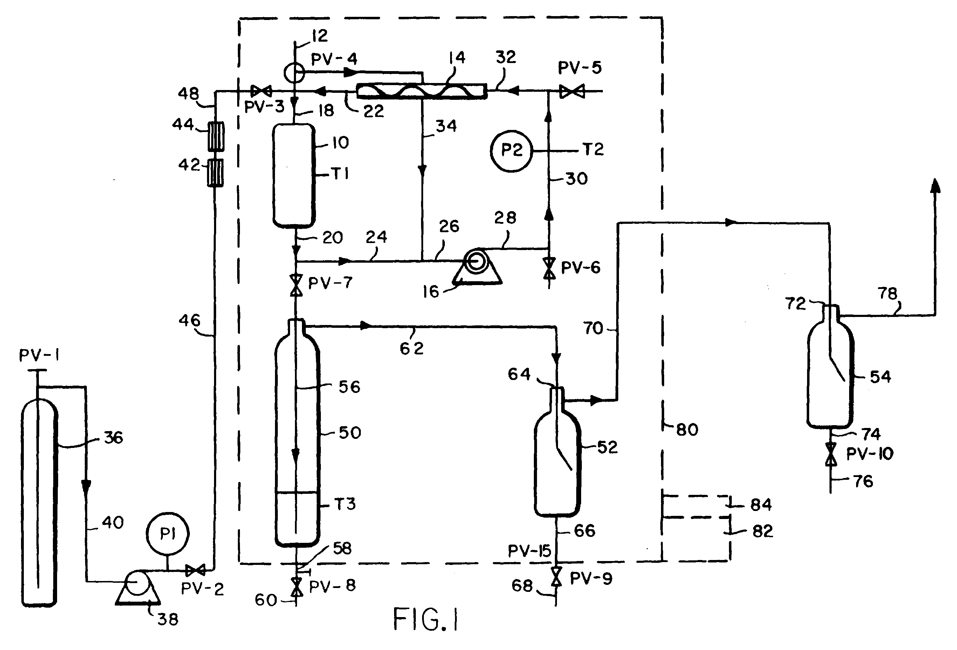

Critical fluid viral inactivation of a proteinaceous product containing viral contaminants can be conducted in an apparatus such as the one shown in Figure 1, or in other variations of the same adapted to permit a critical fluid to be added to a product, adapted to control pressure, temperature, mixing and residence time, and adapted to separate the critical fluid from the virally inactivated proteinaceous product.

Process valves PV-3, PV-5, PV-6 and PV-7 are all 1/4" (0.635cm) full-opening two-way Stainless Steel ball valves rated for 6,000 psig (a gauge pressure of 4.137x10⁷N/m²) at 100°C (Parker, Huntsville, Alabama); PV-4 is a three-way stainless steel ball valve (Parker, Huntsville, Alabama). In some embodiments of the apparatus, process valve PV-7 is a back-pressure regulator (Model No. 26-1722-24, Tescom Corporation, Elk River, Minnesota) with which the operator can control the decompression of the separation chamber and the high pressure circulation loop. The particular soaking chamber 10 employed was a high pressure stainless steel vessel having a capacity of 150 ml and an operating pressure of 5.000 psig (a gauge pressure of 3.447x10⁷N/m²) at 100°C (Model Z148, Hoke Inc., Cresskill, New Jersey). A K-type thermocouple T1 was placed in contact with the outer surface of the soaking chamber 10 and the temperature monitored on a digital temperature-indicator-controller (Model No. CN-310KC, Omega Engineering, Inc., Stamford, Connecticut).

The soaking chamber 10 has a top inlet line 18 and a bottom outlet line 20. The inlet line 18 communicates at a fluid joint with the source of critical fluid, the product injection port 12, and the outlet line 22 of the static in-line mixer 14. The outlet line 20 of the soaking chamber 10 and one end of conduit line 24 are connected at a fluid joint which joint in turn is connected to the decompression valve PV-7. The other end of conduit line 24 is connected at a fluid joint to the inlet line 26 of the circulation pump 16. The particular circulation pump 16 used was a variable speed (0 to 9,000 rpm), high pressure (5,000psig -- a gauge pressure of 3.447x10⁷N/m² -- at 150°C) gear pump capable of a flowrate of 300ml/min at a pressure differential of 10 to 20 psig (a gauge pressure of 6.89x10³ to 1.379x10⁴N/m²)(Modified Model No: 183, custom-built by Micropump,Concord, California). The circulation pump 16 had a cooling head made of an aluminum block connected to a circulating, refrigerated water bath capable of maintaining temperatures as low as 5C and a coaling rate 15,300 Btu/h (1.614x10⁷J/h) (Model No: HX-150, Neslab, Inc., Concord, New Hampshire). The discharge line 28 of circulation pump 16 is connected at a fluid joint to one end of the conduit line 30, which joint in turn is connected to drain valve PV-6. The other end of conduit line 30 is connected at a fluid joint to the inlet line 32 of the static in-line mixer 14 which joint in turn is connected to vent valve PV-5. Conduit 30 has an in-line pressure indicator P-2 and an in-line K-type thermocouple T2 which is connected to a temperature-indicator controller (Model No. CN-310KC, Omega, Stamford, Connecticut).

The particular static -n-line mixer 14 employed was a 3/16" (0.47625cm) ID x 7 1/2" (19.05cm) long x 27 element tube mixer rated for a 4,642 psig (a gauge pressure of 3.201x10⁷N/m²) at 300°F (149°C) (Kenics Mixer Model No: 37-03-075, Chemineer, Dayton, Ohio). The outlet line 22 of the static in-line mixer 14 and the inlet line 18 to the separation chamber 10 are connected at a fluid joint. This joint is connected to critical fluid feed valve PV-3 and also is connected to the three-way process valve PV-4. The three-way process valve PV-4 allows fluid connection between the injection port 12 and either the recycle conduit 34 or the inlet line 18 of the soaking chamber 10. The recycle conduit at its other end is connected to the joint between the conduit line 24 and inlet 26 of the circulation pump 16.

The critical fluid is in fluid communication with feed valve PV-3 via a series of conduit lines interrupted by valves, pumps and filters. Release of critical fluid from the container 36 is controlled by valve PV-1 on the head of the high pressure container. The fluid is conducted from the container 36 to the inlet of compressor 38 via conduit line 40. The particular compressor employed was a single-ended diaphragm compressor which can compress gas or liquid up to 10,000 psig (a gauge pressure of 6.895x10⁷N/m²) at a flow rate of 40 standard litres per minute (Model No. J-46-13411, Superpressure Gas Compressor, Newport Scientific, Jessup, Maryland). A process valve PV-2 is connected to the outlet of the compressor 38 and can be closed when the desired pressure is achieved. The fluid is conducted from the compressor 38 to a pair of in-line filters 42, 44 via conduit line 46. The particular in-line filters used were a 7 micron (70nm) sintered stainless steel cup filter 42 (Model No: SS-8F-K4-7 in a 6TF stainless steel housing, Nupro Company, Willoughby, Ohio) and a 0.5 micron (5nm) sintered stainless steel cup filter 44 (Model No: SS-8F-K4-05 in a 6TF stainless steel housing, Nupro Company, Willoughby, Ohio). The fluid exits the outlet of filter 44 and is conducted via conduit 48 to valve PV-3.

The high pressure recirculation loop interfaces with the product recovery, low pressure half of the apparatus which is made up of a 500 ml decompression chamber 50, a first low pressure trap 52, and several two-way valves and connecting lines. The exhaust system consists of a second low pressure trap 54 leading to a vent line which exhausts to the atmosphere. In other embodiments, the vented critical fluid is first filtered and then recycled to the inlet of the compressor P-1.

The mixture in the soaking chamber 10 can be moved through the outlet line 20 via decompression valve PV-7 to a decompression tube 56 which extends to within about 1/4" (0.635 cm) of the bottom of decompression chamber 50. The decompression chamber 50 has one inlet through which decompression tube 56 is inserted and sealed, and two outlet lines. The bottom outlet line 58 exits the bottom of the chamber 50 and is connected to process valve PV-8 (same type as PV-2) which in turn is connected to a sample port 60 for the recovery of liquid solvents and slurries. The top outlet line 62 exits the top of the decompression chamber 50 and is connected to low pressure trap 52. The particular decompression chamber employed was a 500 ml stainless steel high pressure chamber rated for 5,000 psig (a gauge pressure of 3.447x10⁷N/m²) at 100°C (Model No: Z152, Hoke Inc., Cresskill, New Jersey).

The low pressure trap 52 has one inlet 64 through which the top outlet line 62 of the decompression chamber 50 is inserted and sealed. It also has two outlet lines. The bottom outlet line 66 exits the bottom of the low pressure trap 52 and is connected to process valve PV-9 which in turn is connected to a sample port 68 for the recovery of any liquid solvents and slurries carried over during the decompression process. The top outlet line 70 exits the top of low pressure trap 52 and is connected to a second low pressure trap 54. The second low pressure trap 54 has one inlet 72 through which the top outlet line 70 of low pressure trap 52 is inserted and sealed and two outlet lines. The bottom outlet line 74 exits the bottom of the second low pressure trap 54 and is connected to process valve PV-10 which is connected to a sample port 76 for the recovery of any liquid solvents and slurries carried over during the decompression process. The top outlet line 78 exits the top of second low pressure trap 54 and is vented to atmosphere. The particular low pressure traps employed were 150 ml high pressure Monel chambers rated for 5,000 psig (a gauge pressure of 3.447x10⁷N/m²) at 100°C (Model No: Z152, Hoke Inc., Cresskill, New Jersey).

For reasons of safety and equipment flexibility, the product recovery half of the apparatus was also designed for continuous operation at a pressure of 5,000 psig (a gauge pressure of 3.447x10⁷N/m²) at 100°C. Both the high pressure circulation loop and the product recovery half of the apparatus are enclosed in a polycarbonate (Lexan) box 80 which serves as a containment chamber. This chamber is heated by a 1500W (Pelonis) heater 82 and controlled by a solid state ON-OFF temperature-indicator-controller 84 (Model No. CN-310KC, Omega, Stamford, Connecticut) based on the in-line temperature T2 in conduit 30 attached to the discharge line 28 of the circulation pump P-2 16.

As an initial condition, the system is cleaned, sterilized and dried, and is at operating temperature (room temperature to 40°C) with all process valves (PV) in the closed position.

To accomplish this, the system was rinsed with 0.5mM EDTA in order to complex and remove any metal traces on the inside of the apparatus. The system was sterilized by filling with 70% ethanol solution via the 7 micron (70nm) and the 0.5 micron (5nm) filter elements. The system was then heated to 40°C and the ethanol system was then heated to 40C and the ethanol circulated in the high pressure circulation loop of the apparatus for about 30 minutes. The blowdown valve PV-7 was then opened and the entire system filled with 70% ethanol to the vent valve after LPT-2. All valves, namely PV-4, PV-5, PV-6, PV-8, PV-9 and PV-10, were bled until ethanol was seen. Valves PV-4 and PV-6 were covered with gauze soaked in 70% ethanol and then covered with aluminum foil. The system was held at 40C for approximately 30 minutes. The ethanol was then displaced from the system with pressurized (around 100 psig -- a gauge pressure of 6.89x10⁵N/m² --) and filtered (through the 7 micron -- 70nm -- and 0.5 micron -- 5nm -- filters) nitrogen through valves PV-6, PV-8, PV-9, and PV-10. When liquid was not longer coming out, these valves were all covered with ethanol soaked gauze and aluminium foil. The entire system was then rinsed with 0.2 micron (2nm) filtered distilled and deionized (DDI) water. All valves were bled and the water displaced out of the system using filtered nitrogen under pressure. The sample loading tube, collection flasks and the fill bell (with silicon tubing attached) were all sterilized in an autoclave set at 250°F (121°C) for 30 minutes on fast exhaust. The loading tube was wrapped in gauze; about 2 ml of DDI water (in order to generate steam) was placed in the flasks which had a gauze and cotton plug covered with aluminum foil. When the steam cycle was completed, a 20 minute drying cycle was conducted.

In its normal operating mode, valves PV-4 and PV-5 are opened and an aliquot of test solution is aseptically introduced through the 3-way valve PV-4 using a sterile syringe and a presterilized loading tube. The 3-way valve PV-4 is then turned so that the critical fluid recycle line communicates with the soaking chamber, and the vent valve PV-5 is closed. PV-1 is then opened, supplying the solvent to the compressor P-1. The compressor is turned on and immediately thereafter valves PV-2 and PV-3 are opened, introducing the critical fluid solvent into the high pressure circulation loop. When operating pressure is attained, the compressor is turned off and valve PV-3 is closed.

After system stabilization, the circulation pump P-2 is turned on and its speed adjusted, typically to 75 % of maximum speed or 6,750 rpm. P-2 draws both the proteinaceous product from the bottom of the soaking chamber and the critical fluid phase from the top of the same unit. The mixture is then pumped counter-clockwise, mixed by static in-line mixer and returned to the soaking chamber. After mixing for a defined residence time. P-2 is turned off. The decompression valve PV-7 is then fully opened to depressurize the soaking chamber and the high pressure circulation loop. The rate of decompression was approximately 500 psig (3.45x10⁶N/m²) per second when PV-7 is a 1/4" (0.635cm) ball valve. By using a back-pressure regulator instead of a 1/4" (0.635cm) ball valve as the decompression valve PV-7, the rate of decompression can be controlled. In some experiments, the rate of decompression was controlled to approximately 1,000 psi (6.89x10⁶N/m²) per minute. No differences were observed between rapid (500 psi --3.45x10⁶N/m² -- per second) and slow (1,000 psi --6.89x10⁶N/m² -- per minute) rates of decompression on the impact of critical fluid on viral infectivity and product activity. The experiments in Example 1 below were conducted with slow decompression while the experiments in the remaining examples presented below were conducted with rapid decompression. After decompression, product was aseptically collected into a sterile flask from the decompression chamber 50 through sample port 60 which was aseptically connected to a sterile fill bell. The collected sample was stored on ice until assayed.

In the examples described herein, samples of a recombinant murine C-type retrovirus were prepared as follows. The cell line Psi-2 (Mann et al., 1983, Cell 33:153-159), which produces defective Moloney murine leukemia virus particles (particles that lack viral nucleic acid, but are otherwise wild type), was transfected with plasmid DNA consisting of the retrovirus vector LNCX (Miller and Rosman, 1989, Biotechniques 7:980-990) that had previously been modified by the insertion of a chimeric beta-rubulin gene (Bond et al., 1986, Cell 44:461-468) into the Stu 1 site of the LNCX vector. Clonal cell lines stably expressing the transfected DNA were derived and maintained as virus-producing stocks. By packaging the RNA produced by the transfected DNA into the defective particles, these cells produce infectious particles that are entirely normal with respect to overall structure, stability, and mode of infection, but containing the plasmid-derived rather that the wild type, genetic material (Mann er al., Ibid).

Because the LNCX sequences contain the antibiotic resistance gene neo and a suitable promoter, infection of cells with the recombinant retrovirus confers upon the cells resistance to the antibiotic Geneticin (G418). Conferral of Geneticin-resistance onto cells and their progeny was therefore used as the property by which virus titers were determined. Specifically, virus stocks were prepared by culturing the virus-producing cells at high density (50% confluence) in fresh culture medium (Dulebecco's modified Eagle's medium supplemented with 10% iron-supplemented calf serum [Hyclone, Inc., Logan. UT], glutamine [1 mM], penicillin [100 U/ml] and streptomycin [100 µg/ml]) for 18 hours at 37C in a humidified 5% CO2 atmosphere. Culture medium was harvested and passed through a 0.2 micron filter, divided into aliquots, quick frozen using a dry-ice/ethanol bath and stored at -80°C. Immediately before use, samples were warmed in a 37°C water bath until just thawed and held on ice. Aliquots of a single virus preparation were used for the examples described below.

After virus-containing samples were mixed with other materials and treated as described in each of the examples, they were returned to ice, and assayed within 8 hours. Titer tests were performed by seeding 35 mm-style culture dishes with a mouse fibroblast line (NIH3T3 cells) in 3 ml of the culture medium described above at a density of 10,000 cells per well the evening before the assay was to be performed. Virus samples were added to the culture medium over the cells at various dilution from 10⁰ (undiluted) to 10⁻⁶. In some cases (where indicated), polybrene (Aldrich Chemical Co.) was added along with virus at a final concentration of 2 µg/ml. Polybrene is a polycation that enhances the infectivity of retroviruses up to 100-fold, apparently by enhancing the adsorption of viral particles to cell surfaces; polybrene is commonly used in assaying mouse retrovirus titers. After addition of virus, test cells were returned to the incubator overnight, and then washed into fresh medium containing 1 mg/ml Geneticin. This medium was replenished 2-3 times over the following two weeks, after which the plates of cells were fixed (with 10% formalin in phosphate-buffered saline), stained with Coomassie Blue dye, washed in 25% ethanol, and air-dried. During the two weeks of growth, each virus-infected cell gives rise to a colony of Geneticin-resistant cells. Because the LNCX-genome does not encode the functions necessary to produce new virus particles, virus-infected particles do not spread infection to nearby cells. Therefore, all cells not infected during the initial overnight exposure to the virus remain sensitive to the antibiotic, and die during the two week incubation. Consequently, the number of colonies (as visualized by Coomassie blue staining) present at two weeks provides an accurate reflection of the number of infectious virus particles initially applied to the cells. In parallel with all examples shown, control experiments were performed to demonstrate that cells not exposed to virus produced no colonies at two weeks, and cells exposed to untreated virus produced the expected number of colonies.

As a result of our experiments, including those described in the examples below, we have discovered that critical fluids can be toxic to viruses. The cause of toxicity has not yet been determined but may involve any of at least four possible mechanisms:

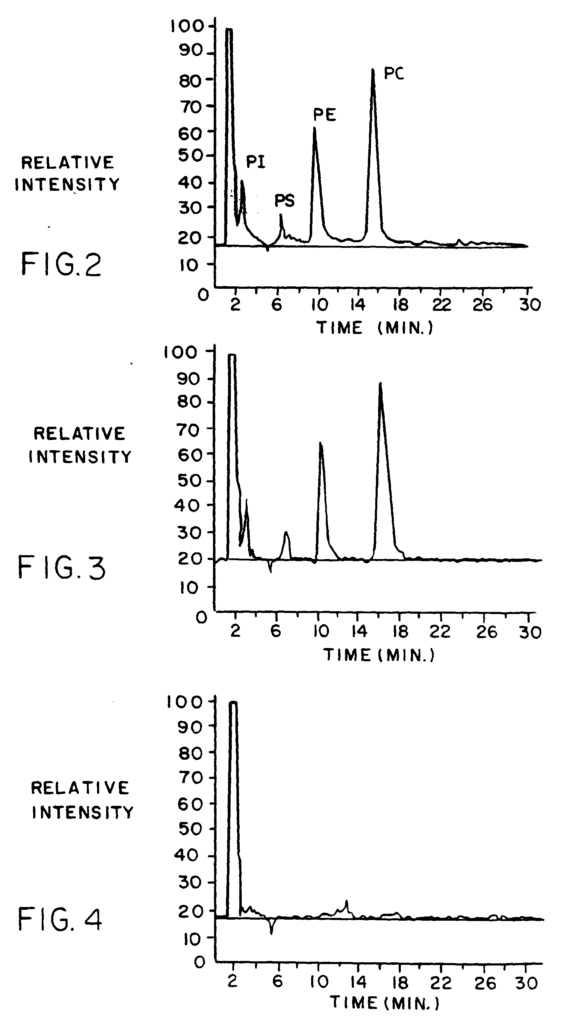

Extraction of Viral Components: Critical fluids are excellent solvents. They can exhibit a liquid-like density and, at the same time, gas-like properties of diffusivity, viscosity and surface tension. The critical fluids may be penetrating cells and subcellular structures (exhibiting solubilization properties similar to those of organic liquids). For example, supercritical carbon dioxide at 3,000 psig (a gauge pressure of 2.068x10⁷N/m²) and 40°C has a density of 0.8 g/cc and behaves like an organic solvent with the diffusivity of a gas. Critical fluids have additional degrees of freedom over organics in that solvation capacity can be readily adjusted by changing density (via changes in temperature and/or pressure), and selectivity can be altered by the type and concentration of polar entrainers such as an alcohol. One virus component that might be disrupted by critical fluids is the viral envelope, which contains cell derived lipids. Preliminary studies with yeast cells (see Figure 2) suggest that critical fluids are indeed very effective at phospholipid extraction from biological samples. Figure 2a shows an HPLC chromatogram of a hexane-isopropanol extract of untreated Baker's yeast feedstock; the major phospholipid peaks are clearly defined, having been identified and quantified by the use of standards. Figure 2c shows that supercritical nitrous oxide (SCF N2O) is very effective in liberating several phospholipids from Baker's yeast. Figure 2b shows that supercritical nitrogen (SCF N2), a control solvent at identical conditions of pressure, temperature and time, is relatively ineffective for the liberation of phospholipids from Baker's yeast.

Explosive Decompression: Upon depressurization, critical fluids exhibit large specific volume changes. For example, supercritical carbon dioxide will expand by a factor of approximately 400 fold when depressurized from 3,000 psig (a gauge pressure of 2.068x10⁷N/m²) and 40°C to atmospheric conditions. When depressurization is sufficiently rapid, explosive decompressive forces may play a role in disrupting microscopic structures. This phenomenon is demonstrated in Figure 3 in which yeast cells were exposed to the critical fluid viral inactivation process, using the apparatus depicted in Figure 1. The top scanning electron micrograph shows a single yeast cell before treatment (a magnification of 15,600X), while the bottom plate shows a yeast cell after treatment (a magnification of 20,600X). Disruption of the normally rigid yeast cell wall is apparent. It is possible that such explosive forces also participate in the inactivation of viruses, which are considerably smaller than yeast, but direct evidence has not yet been obtained.

Virus Particle Disintegration: The components of viruses (proteins, nucleic acid, and sometimes lipid) self-assemble inside cells to produce infectious viral particles. Noncovalent forces of protein aggregation are primarily responsible for holding these particles together. Under conditions of very high pressure (e.g. 20,000 psig -- a gauge pressure of 1.379x10⁸N/m² -- and higher), protein-protein and protein-lipid interactions can be disrupted. Thus, it is possible that viral particles undergo partial or total disassembly under the conditions that we have used.

Chemical Modification: Under atmospheric conditions, most viruses are chemically quite stable. Under conditions of high pressure and in the presence of critical fluids, however, chemical reactions might occur that do not normally do so under atmospheric conditions at a significant rate. Examples of such reactions might be the oxidation of lipids (perhaps catalyzed by nitrous oxide), or the hydrolysis or chemical modification of nucleic acids, both of which might interfere with virus function. Chemical reactions involving proteins might also occur, although the remarkably mild effects of the viral inactivation process on bovine plasma (see below) suggest that proteins are relatively unaffected by the process.

In addition to demonstrating that critical fluids can be toxic to viruses, we have examined the impact of the critical fluid viral inactivation process on biologically active proteins and small molecules. If critical fluid inactivation is to be useful in destroying viruses in biological samples such as blood plasma, cell culture media, pharmaceutical substances, and substances derived using recombinant DNA techniques, the process must be relatively harmless to the beneficial components of those substances. Proteins are of the greatest concern in this regard, because these molecules are susceptible to denaturation by a host of factors including temperature, extremes of pH and ionic strength, drying, exposure to nonaqueous liquids or detergents, and even mechanical shear.

During the conduct of research on several other bioseparation processes utilizing critical fluids, we have subjected several natural and recombinant-DNA proteins to a variety of critical fluids under different conditions. In one particularly revealing study conducted in collaboration with a biopharmaceutical company, a recombinant-DNA growth hormone of commercial importance was treated with supercritical fluids and its activity measured by a very precise HPLC technique (which is approved by the FDA and the WHO). Our data indicates that there were no detectable changes in activity and structural conformation of the protein tested (under conditions which are very similar to those in the examples given below). We have observed that supercritical carbon dioxide is not a good candidate for liquid media systems because of acid pH effects. Other than this negative impact on proteins, we have not detected any significant changes in biological activity by critical fluids over a wide range of pressure (0 to 10,000 psig -- a gauge pressure of 6.89x10⁷N/m² --).

In order to demonstrate that the conditions necessary for viral inactivation are not exceedingly harmful to the beneficial constituents of proteinaceous products, several experiments were conducted and are reported in Examples 1, 2, 3 and 4 below. Similar conditions were then utilized to inactivate viral particles in the presence of different levels of proteins; these experiments are reported in Examples 5, 6 and 7 below.

Examples of our disclosure are given below to show how variables such as critical fluid type, cosolvent concentration, temperature, pressure and time can effect reduction in viral activity and biological activity. It should be understood that the critical fluid viral inactivation process is not limited to the following examples which are presented solely to further illustrate the invention.

### EXAMPLE 1

### IMPACT OF SUPERCRITICAL CARBON DIOXIDE AND NITROUS OXIDE ON BOVINE PLASMA

In order to evaluate if critical fluid treatment would adversely affect the beneficial constituents of plasma, several experiments were carried out with bovine plasma. Aseptically collected bovine blood treated with an anticoagulant (sodium citrate) was centrifuged at a speed of 3,000 rpm for 30 minutes in a refrigerated centrifuge at 10°C. 75 ml of the decanted plasma was introduced into the apparatus shown as Figure 1, contacted with the critical fluid at the conditions listed in the top half of Table 1 and then slowly decompressed to atmospheric conditions.

In this series of experiments, there were two controls -- CFI-0 which is the untreated, unprocessed plasma, and CFI-3 which is processed in the critical fluid viral inactivation (CFI) apparatus without critical fluids. The latter control accounts for the mechanical impact of mixing on proteins and other beneficial constituents. The recovered samples were spun down in a refrigerated centrifuge at 3.000 rpm for 30 minutes at 10°C; the clarified plasma was then sent out to Tufts University Veterinary Diagnostic Laboratory. Boston. MA for analysis. The results of this analysis, summarized in the bottom half of Table 1, indicate that supercritical carbon dioxide had an adverse impact on the beneficial constituents of the bovine plasma, namely alkaline phosphatase (AKP), lactic dehydrogenase (LDH) and creatine phosphokinase (CPK), while the impact of supercritical nitrous oxide was much less severe.

**TABLE 1**

| **IMPACT OF CRITICAL FLUID CARBON DIOXIDE AND NITROUS OXIDE ON BOVINE PLASMA** | | | | |
|---|---|---|---|---|
| PARAMETER | CFI-0 | CFI-1 | CFI-2 | CFI-3 |
| Critical Fluid | --- | CO2 | N20 | --- |
| Pressure (psig) | --- | 4,000 | 4,000 | --- |
| Temperature (C) | 4 | 40 | 40 | 40 |
| Time (mins) | --- | 30 | 30 | 30 |
| Glucose (mg/dl) | 64 | 41 | 62 | 60 |
| BUN (mg/dl) | 17 | 17 | 17 | 18 |
| Protein (g/dl) | 6.9 | 5.1 | 6.5 | 6.7 |
| AKP (U/l) | 252 | 0 | 178 | 228 |
| LDH (U/l) | 1,458 | 38 | 1,011 | 1,428 |
| Albumin (g/dl) | 3.4 | 5.5 | 4.2 | 3.2 |
| CPK (U/l) | 547 | 44 | 199 | 432 |
| BUN - Blood Urea Nitrogen AKP - Alkaline Phosphatase LDH - Lactic Dehydrogenase CPK - Creatine Phosphokinase 1 psig equals a gauge pressure of 6.89x10³N/m². | | | | |

### EXAMPLE 2

### IMPACT OF PRESSURE ON CRITICAL FLUID NITROUS OXIDE ON BOVINE PLASMA

The second set of runs was conducted with supercritical nitrous oxide at different pressures. Within 24 hours after treatment, a SMAC analysis was performed on the recovered plasma by Bioran Laboratories, Cambridge, MA. All SMAC results were repeated and verified by Bioran Laboratories. The conditions of these runs and the results of the analysis are listed in Table 2. At the end of the experiments, the mixture of critical fluid and bovine plasma was rapidly decompressed (at a rate around 100 psig/sec) and the critical fluid separated from the now treated plasma. The results listed in Table 2 indicate that supercritical nitrous oxide had little or no impact on blood urea nitrogen (BUN), AKP, LDH, albumin, triglycerides and cholesterol over the range of pressures tested, and there was little or no sensitivity to the level of pressure between 1,000 (a gauge pressure of 6.89x10⁶N/m²) and 3,000 psig (a gauge pressure of 2.068x10⁷N/m²). It should be noted that the glucose values for experiments CFI-4 to CFI-7 are about twice that of the control CFI-00. These results are difficult to fathom since the glucose level of bovine plasma treated with SCF N20 at 4,000 psig (a gauge pressure of 2,758x10⁷N/m²) and 40°C was about the same as the control in the first example (see Table 1). The higher glucose levels could have been caused by the critical disruption of red blood cells which were not removed in the plasma preparation.

**TABLE 2**

| **IMPACT OF PRESSURE ON CRITICAL FLUID NITROUS OXIDE ON BOVINE PLASMA** | | | | | |
|---|---|---|---|---|---|
| PARAMETER | CFI-00 | CFI-4 | CFI-5 | CFI-6 | CFI-7 |
| Critical Fluid | --- | N2O | N2O | N2O | N2O |
| Pressure (psig) | --- | --- | 1,000 | 2,000 | 3,000 |
| Temperature (C) | 4 | 40 | 40 | 40 | 40 |
| Time (mins) | --- | 30 | 30 | 30 | 30 |
| Glucose (mg/dl) | 77 | 148 | 149 | 155 | 153 |
| BUN (mg/dl) | 18 | 16 | 15 | 16 | 16 |
| Protein (g/dl) | 7.7 | 6.3 | 5.9 | 6.4 | 6.5 |
| AKP (U/l) | 24 | 30 | 30 | 34 | 35 |
| LDH (U/l) | 1.111 | 891 | 767 | 883 | 891 |
| Albumin (g/dl) | 2.5 | 2.4 | 2.4 | 2.6 | 2.5 |
| Triglycerides (mg/dl) | 11 | 16 | 17 | 18 | 17 |
| Cholesterol (mg/dl) | 143 | 148 | 136 | 150 | 154 |
| BUN - Blood Urea Nitrogen AKP - Alkaline Phosphatase LDH - Lactic Dehydrogenase CPK - Creatine Phosphokinase 1 psig equals a gauge pressure of 6.89x10³N/m². | | | | | |

### EXAMPLE 3

### IMPACT OF CRITICAL FLUID TYPE ON BOVINE PLASMA

In this series of experiments, the critical fluid was varied; with the exception of the control --CFI-00 -- process conditions were 3,000 psig (a gauge pressure of 2.068x10⁷N/m²), 40°C and 30 minutes. The critical fluids tested were nitrous oxide (N2O), ethylene (C2H4), ethane (C2H6), propane (C3H8), tri-fluoromethane (CHF3 or Fr-23), chlorodifluoromethane (CHClF2 or Fr-22). The conditions of these experiments and some relevant thermo-dynamic properties are listed in the top half of Table 3. These solvents, with the exception of propane and Freon-22, are all supercritical at the tested conditions of 3,000 psig (a gauge pressure of 2.068x10⁷N/m²) and 40°C. The results of explosive decompression experiments with these critical fluids and bovine plasma are listed in the bottom half of Table 3. These results can be compared against values for the control in Table 2. Apart from the doubling of glucose concentration levels and some detrimental effects on LDH in CFI-7 to CFI-12, the first six critical fluids tested in Table 3 had a negligible impact on blood constituents. Near-critical Freon-22 and supercritical Freon-23 had the most significant impact on LDH decreasing concentration by slightly more than 50% of the original value.

**TABLE 3**

| **IMPACT OF CRITICAL FLUID TYPE ON BOVINE PLASMA** | | | | | | | |
|---|---|---|---|---|---|---|---|
| PARAMETER | CFI-7 | CFI-8 | CFI-9 | CFI-10 | CFI-11 | CFI-12 | CFI-13 |
| Critical Fluid | N20 | C2H4 | C2H6 | C3H8 | Fr-23 | Fr-22 | N2 |
| Crit. Press. (psia) | 1,051 | 731 | 709 | 616 | 701 | 722 | 493 |
| Crit. Temp.(C) | 36.4 | 9.2 | 32.2 | 96.6 | 25.9 | 96.0 | -147.0 |
| Pressure (psig) | 3,000 | 3,000 | 3,000 | 3,000 | 3,000 | 3,000 | 3,000 |
| Temperature (C) | 40 | 40 | 40 | 40 | 40 | 40 | 40 |
| Time (mins) | 30 | 30 | 30 | 30 | 30 | 30 | 30 |
| Glucose (mg/dl) | 153 | 157 | 156 | 161 | 166 | 132 | 68 |
| BUN (mg/dl) | 16 | 17 | 16 | 17 | 16 | 16 | 12 |
| Protein (g/dl) | 6.5 | 6.8 | 6.9 | 7.1 | 6.4 | 6.5 | 6.6 |
| AKP (U/l) | 35 | 34 | 33 | 35 | 26 | 24 | 24 |
| LDH (U/l) | 891 | 808 | 837 | 907 | 522 | 510 | 971 |
| Albumin (g/dl) | 2.5 | 2.7 | 2.5 | 2.6 | 2.7 | 2.8 | 2.3 |
| Triglycerides (mg/dl) | 17 | 19 | 18 | 18 | 30 | 29 | 11 |
| Cholesterol (mg/dl) | 154 | 154 | 152 | 152 | 124 | 134 | 128 |
| BUN - Blood Urea Nitrogen AKP - Alkaline Phosphatase LDH - Lactic Dehydrogenase CPK - Creatine Phosphokinase 1 psia equals an absolute pressure of 6.89x10³N/m². 1 psig equals a gauge pressure of 6.89x10³N/m². | | | | | | | |

### EXAMPLE 4

### IMPACT OF RESIDENCE TIME AND OPERATING TEMPERATURE ON THE CRITICAL FLUID TREATMENT OF BOVINE PLASMA

The impact of residence time and operating temperature on some of the beneficial constituents of bovine plasma treated by SCF N20 at 3,000 psig (a gauge pressure of 2.068x10⁷N/m²) is listed in Table 4. The data suggests that temperature more than time had an impact on SCF N2O treated bovine plasma. For example, both CFI-14 and CFI-15 which were conducted at 30°C had negligible impacts on glucose, total protein and LDH versus CFI-7 which was conducted at 40°C. Also, there was no significant difference in the impact of CFI-14 and CFI-15 which had residence times of 30 and 5 minutes.

**TABLE 4**

| **IMPACT OF RESIDENCE TIME AND OPERATING TEMPERATURE ON THE CRITICAL FLUID TREATMENT OF BOVINE PLASMA** | | | | |
|---|---|---|---|---|
| PARAMETER | CFI-00 | CFI-7 | CFI-14 | CFI-15 |
| Critical Fluid | --- | N2O | N2O | N20 |
| Pressure (psig) | --- | 3,000 | 3,000 | 3.000 |
| Temperature (C) | 4 | 40 | 30 | 30 |
| Time (mins) | --- | 30 | 30 | 5 |
| Glucose (mg/dl) | 77 | 153 | 74 | 74 |
| BUN (mg/dl) | 18 | 16 | 14 | 17 |
| Protein (g/dl) | 7.7 | 6.5 | 7.3 | 7.2 |
| AKP (U/l) | 24 | 35 | 30 | 29 |
| LDH (U/l) | 1,111 | 891 | 1,018 | 1.057 |
| Albumin (g/dl) | 2.5 | 2.5 | 2.5 | 2.6 |
| Triglycerides (mg/dl) | 11 | 17 | 13 | 10 |
| Cholesterol (mg/dl) | 143 | 154 | 134 | 137 |
| BUN - Blood Urea Nitrogen AKP - Alkaline Phosphatase LDH - Lactic Dehydrogenase CPK - Creatine Phosphokinase 1 psig equals a gauge pressure of 6.89x10⁶N/m². | | | | |

### EXAMPLE 5

### IMPACT OF RESIDENCE TIME ON CRITICAL FLUID VIRAL INACTIVATION OF MURINE-C RETROVIRUS IN CULTURE MEDIUM

Several tests were conducted with 30 ml of culture medium containing murine C-type retroviruses and supercritical nitrous oxide at approximately 3,000 psig (a gauge pressure of 2.068x10⁷N/m²) and 40°C. These experiments (CFI-18, CFI-19, and CFI-20 listed in Table 5) were conducted at different residence times ranging from 5 to 121 minutes; CFI-21 in Table 5 was conducted with supercritical nitrogen at similar conditions of temperature and pressure for 30 minutes. Between each run, and as described above, the experimental apparatus was rinsed with sterile deionized water, sterilized with 70 % ethanol through 7 micron (70nm) and 0.5 micron (5nm) filters, and again rinsed with sterile deionized, distilled water; the apparatus was then dried with filtered compressed nitrogen or air.

After each experiment, the recovered samples were halved; one half was spun down at 2,500 rpm for 10 minutes and the supernatant subjected to 0.45 micron (45nm) filtration. Titer tests, ability to infect 3T3 fibroblasts and confer G418 resistance, were conducted on duplicate 2.3 ml samples at six different dilutions. The appropriate titers of recovered samples are listed in Table 5 and shown as a function of residence time in Figure 4. The results indicate that supercritical nitrogen was relatively ineffective in inactivating the retrovirus whereas supercritical nitrous oxide was very effective in inactivating the retrovirus. The supercritical nitrogen results can be correlated with its inability to solubilize/liberate phospholipids as shown in Figure 3. On the other hand, supercritical nitrous oxide rapidly inactivated the retrovirus in a period between 30 and 121 minutes.

**TABLE 5**

| **IMPACT OF RESIDENCE TIME ON CRITICAL FLUID VIRAL INACTIVATION OF MURINE-C RETROVIRUS IN CULTURE MEDIUM** | | | | | |
|---|---|---|---|---|---|
| PARAMETER | Control | CFI-18 | CFI-19 | CFI-20 | CFI-21 |
| Critical Fluid | --- | N2O | N2O | N2O | N2 |
| Pressure (psig) | --- | 2,560 | 2,690 | 3,180 | 3,250 |
| Temperature (C) | 4 | 43 | 41 | 41 | 41 |
| Time (mins) | --- | 30 | 5 | 121 | 30 |
| Titer (cfu/2.3 ml) | 5,000 | 10 | 50 | < 5* | 3,000 |
| - log10 Reduction | N/A | 2.7 | 2.0 | > 3.0 | 0.2 |

| | | | | | |
|---|---|---|---|---|---|
| * Detection limit | | | | | |
| 1 psig equals a gauge pressure of 6.89x10³N/m². cfu means "colony forming unit". | | | | | |

### EXAMPLE 6

### IMPACT OF CRITICAL FLUID TYPE AND COSOLVENT ON THE VIRAL INACTIVATION OF MURINE-C RETROVIRUS IN SERUM

Several critical fluid viral inactivation experiments were conducted with murine-C retrovirus in serum. Proteins are known to have a protective effect on the viability of viruses. These experiments were thus conducted to evaluate the effectiveness of the critical fluid viral inactivation technique in a protein-rich medium. Murine-C retrovirus in culture medium was mixed 50/50 with serum (serum was a 50/50 mix of Hyclone fetal bovine serum and Hyclone iron supplemented calf serum) and divided into 5 equal aliquots. Experiments were conducted with several critical fluids -- nitrous oxide (N2O), a nitrous oxide/2 mole % ethanol mixture, chlorodifluoromethane (CHClF2 or Fr-22) and propane (C3H8) at approximately 3,000 psig (a gauge pressure of 2.068x10⁷N/m²) and 40°C for a residence time of 30 minutes. One critical fluid experiment (CFI-26) was conducted at 60°C. These experiments are listed in Table 6. Several controls were conducted on samples which were not treated with critical fluids -- one at 40°C, one at 40°C with 2 mole % ethanol in the serum, and one at 60°C.

Titers were run on controls and recovered samples without polybrene enhancement (in duplicate at six dilutions) and with polybrene enhancement (in duplicate at four dilutions). The titer results indicated that 2 mole % ethanol does not affect the titer but that heating at 60°C does destroy the retrovirus. The titer results presented in Table 6 are for polybrene enhanced determinations (these valves are consistent with titers determined without polybrene). The 40°C control had a titer of 65,000 colony forming units/2.3ml. The results listed in Table 6 indicate that the presence of protein did reduce the effectiveness of supercritical nitrous oxide by one to two orders of magnitude (compare CFI-27 in Table 6 to CFI-18 in Table 5). Also, 2 mole % ethanol had little or no impact on the effect of supercritical nitrous oxide. Freon-22, however, had a significant impact on murine-C retrovirus, reducing viral activity by some 3.2 log orders of magnitude after 30 minutes of contacting the serum.

**TABLE 6**

| **IMPACT OF CRITICAL FLUID TYPE AND COSOLVENT ON THE VIRAL INACTIVATION OF MURINE-C RETROVIRUS IN SERUM** | | | | | | |
|---|---|---|---|---|---|---|
| PARAMETER | Control | CFI-26 | CFI-27 | CFI-28 | CFI-29 | CFI-30 |
| Critical Fluid | --- | N2O | N2O | N2O/EtOH | Fr-22 | C3H8 |
| Pressure (psig) | --- | 3,000 | 3,000 | 3,000 | 3,000 | 3,000 |
| Temperature (C) | 40 | 60 | 40 | 41 | 41 | 41 |
| Time (mins) | --- | 30 | 30 | 30 | 30 | 30 |
| Titer (cfu/2.3 ml) | 65,000 | < 5* | 3,000 | 5,000 | 40 | 2,500 |
| - log10 Reduction | N/A | > 4.1 | 1.4 | 1.1 | 3.2 | 1.4 |

| | | | | | | |
|---|---|---|---|---|---|---|
| * Detection limit | | | | | | |
| 1 psig equals a gauge pressure of 6.89x10³N/m². cfu means "colony forming unit". | | | | | | |

### EXAMPLE 7

### IMPACT OF RESIDENCE TIME, CRITICAL FLUID TYPE AND PRESSURE ON THE VIRAL INACTIVATION OF MURINE-C RETROVIRUS IN SERUM

Based on the results in Example 6, several experiments were conducted with Freon-22 at approximately 3,000 psig (a gauge pressure of 2.068x10⁷N/m²) and 40°C to determine the impact of residence time on viral reduction capability. The experiments, listed in Table 7, indicate that Freon-22 can, within the detection limits of the assay, eliminate viral activity within five minutes.

This example also indicates that pressure has a significant impact on the effectiveness of critical fluid viral inactivation. CFI-34 in Table 7 indicate that supercritical nitrous oxide at 5,000 psig (a gauge pressure of 3.447x10⁷N/m²) and 40°C for a residence time of 30 minutes inactivated the entire viral population (within the detection limits of the assay), while CFI-27 in Table 6 shows that supercritical nitrous oxide at 3,000 psig (a gauge pressure of 2.068x10⁷N/m²) and 40°C for a residence time of 30 minutes reduces virus activity by only 1.4 logs.

**TABLE 7**

| **IMPACT OF RESIDENCE TIME, CRITICAL FLUID TYPE AND PRESSUREON THE VIRAL INACTIVATION OF MURINE-C RETROVIRUS IN SERUM** | | | | | | |
|---|---|---|---|---|---|---|
| PARAMETER | Control | CFI-31 | CFI-32 | CFI-33 | CFI-34 | CFI-35 |
| Critical Fluid | --- | Fr-22 | Fr-22 | Fr-22 | N2O | N2 |
| Pressure (psig) | --- | 3,000 | 3,000 | 3,000 | 5,000 | 3,000 |
| Temperature (C) | 40 | 40 | 40 | 41 | 41 | 41 |
| Time (mins) | --- | 5 | 15 | 60 | 30 | 60 |
| Titer (cfu/2.0 ml) | 20,000 | < 5* | 5 | < 5* | < 5* | 3,750 |
| -log10 Reduction | N/A | > 3.6 | 3.6 | > 3.6 | > 3.6 | 0.7 |

| | | | | | | |
|---|---|---|---|---|---|---|
| * Detection limit | | | | | | |
| 1 psig equals a gauge pressure of 6.89x10³N/m². cfu means "colony forming unit". | | | | | | |

## Claims

1. A method of rendering a virus associated with a material inactive characterized in comprising the steps of:
a) contacting the material with a critical or near critical fluid at a pressure of at least 1000 psig (a gauge pressure of 6.89x10⁶N/m²), more preferably about 3000 psig (a gauge pressure of 2.068x10⁷N/m²), said critical or near critical fluid being capable of being received by said virus and being such that, upon removal of the critical or near critical fluid, said virus is inactivated; and
b) removing said critical or near critical fluid from said material and said virus.

2. A method according to Claim 1, wherein said material is a biological material.

3. A method according to Claim 1, wherein said material is a liquid, and said contacting step comprises forming an admixture of critical or near critical fluid and said material.

4. A method according to Claim 1, wherein said material comprises a biological substance selected from proteins, nucleic acid, and biologically active small molecules.

5. A method according to Claim 4, further comprising the step of isolating said biological substance.

6. A method according to Claim 1, wherein said critical or near critical fluid is selected from one or more of fluorocarbons, alkanes and binary fluids.

7. A method according to Claim 6, wherein said critical or near critical fluid is selected from chlorodifluoromethane and trifluoromethane.

8. A method according to Claim 6, wherein said critical or near critical fluid is selected from one or more of ethylene, propane and ethane.

9. A method according to Claim 6, wherein said critical or near critical fluid is selected from one or more of nitrous oxide, nitrogen, and carbon dioxide.

10. A method according to Claim 1, wherein said critical or near critical fluid has a temperature in the range of approximately 0°C to 100°C.

11. A method according to Claim 1, wherein said critical or near critical fluid has a temperature in the range of approximately 4°C to 40°C.

12. A method according to Claim 1, wherein said critical or near critical fluid has a reduced pressure (Pr) having a relationship with the operating pressure and the critical pressure as defined by the equation:$\text{Pr =} \frac{\text{Po}}{\text{Pc}} \text{= X}$ wherein X is a number in the range of 0.2 to 20.0;
Pr is the reduced pressure;
Po is the operating pressure; and
Pc is the critical pressure.

13. A method according to Claim 12, wherein X is a number in the range of 0.5 to 10.0.

## Patentansprüche

1. Verfahren, um einen Virus in Verbindung mit einem Material inaktiv zu machen, dadurch gekennzeichnet, daß es die folgenden Schritte umfaßt:
a) Kontaktieren des Materials mit einem kritischen oder nahe kritischen Fluid mit einem Druck von wenigstens 1000 psig (einem Manometerdruck von 6,89 x 10⁶ N/m²), bevorzugterweise von etwa 3000 psig (einem Manometerdruck von 2,068 x 10⁷ N/m²), wobei das kritische oder nahe kritische Fluid von dem genannten Virus aufgenommen werden kann und derartig ist, daß nach dem Entfernen des kritischen oder nahe kritischen Fluids der genannte Virus inaktiviert wird; und
b) Entfernen des genannten kritischen oder nahe kritischen Fluids von dem genannten Material und dem genannten Virus.

2. Verfahren nach Anspruch 1, bei dem das genannte Material ein biologisches Material ist.

3. Verfahren nach Anspruch 1, bei dem das genannte Material eine Flüssigkeit ist und der genannte Kontaktierungsschritt die Bildung eines Gemisches aus kritischem oder nahe kritischem Fluid und dem genannten Material umfaßt.

4. Verfahren nach Anspruch 1, bei dem das genannte Material eine biologische Substanz umfaßt, ausgewählt aus Proteinen, Nukleinsäuren und biologisch aktiven kleinen Molekülen.

5. Verfahren nach Anspruch 4, ferner umfassend den Schritt des Isolierens der genannten biologischen Substanz.

6. Verfahren nach Anspruch 1, bei dem das genannte kritische oder nahe kritische Fluid ausgewählt wird aus Fluorkohlenwasserstoffen, Alkanen und binären Fluiden oder Kombinationen davon.

7. Verfahren nach Anspruch 6, bei dem das genannte kritische oder nahe kritische Fluid ausgewählt wird aus Chlordifluormethan und Trifluormethan.

8. Verfahren nach Anspruch 6, bei dem das genannte kritische oder nahe kritische Fluid ausgewählt wird aus Ethylen, Propan und Ethan oder Kombinationen davon.

9. Verfahren nach Anspruch 6, bei dem das genannte kritische oder nahe kritische Fluid ausgewählt wird aus Distickstoffoxid, Stickstoff und Kohlendioxid oder Kombinationen davon.

10. Verfahren nach Anspruch 1, bei dem das genannte kritische oder nahe kritische Fluid eine Temperatur im Bereich von etwa 0°C bis 100°C hat.

11. Verfahren nach Anspruch 1, bei dem das genannte kritische oder nahe kritische Fluid eine Temperatur im Bereich von etwa 4°C bis 40°C hat.

12. Verfahren nach Anspruch 1, bei dem das genannte kritische oder nahe kritische Fluid einen reduzierten Druck (Pr) mit einer Beziehung zwischen dem Betriebsdruck und dem kritischen Druck gemäß der folgenden Gleichung hat:$\text{Pr =} \frac{\text{Po}}{\text{Pc}} \text{= X}$ wobei X eine Zahl im Bereich von 0,2 bis 20,0 ist;
Pr der reduzierte Druck ist;
Po der Betriebsdruck ist; und
Pc der kritische Druck ist.

13. Verfahren nach Anspruch 12, bei dem X eine Zahl im Bereich von 0,5 bis 10,0 ist.

## Revendications

1. Un procédé de désactivation d'un virus associé à un matériau inactif, caractérisé en ce qu'il englobe les étapes suivantes :
a) mise en contact du matériau avec un fluide critique ou quasi critique à une pression minimum de 1000 psig (une pression manométrique de 6,89x10⁶ N/m²), plus préférentiellement d'environ 3000 psig (une pression manométrique de 2,068x10⁷ N/m²), ledit fluide critique ou quasi critique étant capable d'être reçu par ledit virus et étant tel que, sur élimination du fluide critique ou quasi critique, ledit virus est désactivé ; et
b) extraction dudit fluide critique ou quasi critique hors dudit matériau et dudit virus.

2. Un procédé selon la Revendication 1, selon lequel ledit matériau est un matériau biologique.

3. Un procédé selon la Revendication 1, selon lequel ledit matériau est un liquide, et ladite étape de mise en contact englobe la formation d'un mélange de fluide critique ou quasi critique et dudit matériau.

4. Un procédé selon la Revendication 1, selon lequel ledit matériau englobe une substance biologique sélectionnée parmi : protéines, acide nucléique et petites molécules actives biologiquement.

5. Un procédé selon la Revendication 4, englobant de plus l'étape d'isolement de ladite substance biologique.

6. Un procédé selon la Revendication 1, selon lequel ledit fluide critique ou quasi critique est sélectionné parmi un ou davantage de : fluorocarbures, alcanes et fluides binaires.

7. Un procédé selon la Revendication 6, selon lequel ledit fluide critique ou quasi critique est sélectionné parmi : chlorodifluorométhane et trifluorométhane.

8. Un procédé selon la Revendication 6, selon lequel ledit fluide critique ou quasi critique est sélectionné parmi un ou davantage de : éthylène, propane et éthane.

9. Un procédé selon la Revendication 6, selon lequel ledit fluide critique ou quasi critique est sélectionné parmi un ou davantage de : oxyde azoté, azote et gaz carbonique.

10. Un procédé selon la Revendication 1, selon lequel ledit fluide critique ou quasi critique a une température dans la plage d'environ 0°C à 100°C.

11. Un procédé selon la Revendication 1, selon lequel ledit fluide critique ou quasi critique a une température dans la plage d'environ 4°C à 40°C.

12. Un procédé selon la Revendication 1, selon lequel ledit fluide critique ou quasi critique a une pression réduite (Pr) étant en relation avec la pression de service et la pression critique telle que définie par l'équation :$\text{Pr =} \frac{\text{Po}}{\text{Pc}} \text{= X}$ dans laquelle
X est un chiffre dans la plage de 0,2 à 20,0 ;
Pr est la pression réduite ;
Po est la pression de service ; et
Pc est la pression critique.

13. Un procédé selon la Revendication 12, selon lequel X est un chiffre dans la plage de 0,5 à 10,0.
